# EUROPEAN PATENT APPLICATION

(11) **EP 2 735 289 A1**
(43) Date of publication of application: **28.05.2014**
(21) Application number: 13193321.0
(22) Date of filing: 18.11.2013
(51) Int. Cl.: A61F 5/01, A61F 5/058

(54) **Improvements in and relating to dynamic splints**

(30) Priority: 21.11.2012 GB 201220914
(71) Applicant: Dynamic Applied Technology Ltd, Halsall Lancashire L39 8RJ (GB)
(72) Inventor: Jackson, Edward Jr, Halsall, GB L39 8RJ (GB); Jackson, Edward Sr, Southport, Merseyside PR8 4SA (GB)
(74) Representative: Hutchinson, Thomas Owen

(57) **Abstract**

An outrigger (100) suitable for use with a dynamic splint (200), the outrigger (100) comprising a support (102), a lever (124) pivotally connected to the support (102), and spring means (40) cooperating between the support (102) and the lever (124) to urge the lever (124) to pivot towards a first position, wherein the outrigger (100) further comprises means (20) for adjustably affixing the support (102) to a mounting assembly (60). The spring means (106) can comprise a coil spring (40, 106) or a cam (78) and cam follower (80) arrangement and the location of the pivot (32) between the lever (124) and support (102) and a joint of a user is suitably configurable, via adjustment of the location of the outrigger (100) on the mounting assembly (60) to provide a substantially constant restoring force to an affected body part throughout a range of movement of the splint (200). The splint (200) can be used to treat a range of body parts, including finger joints, knee joints, ankle joints, etc..

## Description

This invention relates to splints, and in particular, but without limitation, to dynamic hand, wrist and arm splints.

A splint is often used to support a joint following an injury, illness or surgical procedure. Static splints are used to immobilise a joint during the healing process, whereas dynamic splints provide support whilst allowing a certain amount of movement of the joint. Dynamic splints can also be used for rehabilitation purposes, that is to say, to encourage particular joint positions and/or to provide an exercise regime to aid the healing process.

People who have suffered strokes, or who have undergone hand surgery, often develop a clawed hand, that is to say, where the fingers curl up to, or towards a fist position, which can render the hand useless. In such situations, clawing can be corrected using a static splint that restrains the fingers at extension, that is to say, extending straight from the palm of the hand. Immobilising the fingers thus can be painful over extended periods of time, and also prevents the patient from being able to grip objects because the fingers of the affected hand are unable to bend.

To address these issues, but to nevertheless encourage the fingers towards extension, a range of known dynamic splints are available, which comprise springs and/or elasticated straps running along the tops of the patient's fingers, that pull the fingers towards extension, but which nevertheless allow the fingers to be bent by overcoming the spring force. Dynamic splints such as these suffer from the disadvantage of the resilient spring force increasing with increasing finger flexion, which can become tiring, or in the worst cases, causing potentially harmful snap-back as the fingers are relaxed.

A known way of solving this problem is to provide the dynamic splint fitted with outriggers around which the springs or elasticated straps pass. The provision of outriggers essentially elongates the springs/elastic straps, such that the change in spring force for a given change in finger flexion is reduced.

For the avoidance of doubt or confusion, the term "outrigger" used herein means an attachment for a hand, or any other type of, splint that permits a joint being treated to be placed in elastic traction, which definition is known, for example, from the Taber's Cyclopaedic Medical Dictionary (2009, issue 21, p.1655).

Another known type of dynamic split, such as that described in granted European Patent No: 1071385 develops the use of outriggers by providing moveable outriggers that are arranged to pivot about a point that is offset from the centre of movement of the joint in question. By such a configuration, although the actual spring force increases with joint flexion, the provision of a pivoting outrigger enables the force felt by the joint to be resolved in such a way that the force experienced by the finger during flexion either remains substantially the same, or reduces with increasing finger flexion. Such an arrangement can allow a dynamic splint to be used for extended periods of time, allows a user to have use of his or her hand without excessive fatigue, and alleviates the problem of snap back because the restoring force on the fingers does not increase towards maximum flexion.

A known drawback of dynamic splints, such as that described in EP1071385 is the time and complexity of setting them up for individual patients because the biomechanics require the: spring tension; outrigger pivot point, position relative to the affected joint and length; support and other factors to be customised for each patient. For example, each patient needs a support plate to be moulded to fit their hand/wrist, and the length of the pivoting outriggers needs to be selected to correspond to a certain ratio of joint spacing, the outriggers need to be positioned so that they align correctly with the affected joint in question and the springs need to be appropriately selected and setup so that they act in an appropriate direction relative to the joint and outrigger axes. It is understood that a typical initial casting and set-up can take from one to several hours per patient. Therefore, whilst dynamic splints of this type offer considerable flexibility in terms of fitting and set-up, they can, nevertheless, be time consuming and difficult to set-up.

A need therefore arises for a solution to one or more of the above problems.

According to a first aspect of the invention, there is provided an outrigger suitable for use with a dynamic splint, the outrigger comprising a support, a lever pivotally connected to the support, and spring means cooperating between the support and the lever to urge the lever to pivot towards a first position, wherein the outrigger further comprises means for adjustably affixing the support to a mounting assembly.

According to a second aspect of the invention, there is provided a dynamic splint comprising a mounting assembly, and at least one outrigger adjustably affixed to the mounting assembly, wherein the outrigger comprises a support, a lever pivotally connected to the support, and spring means cooperating between the support and the lever to urge the lever to pivot towards a first position.

According to a third aspect of the invention, there is provided mounting assembly for a dynamic splint comprising means for adjustably affixing an outrigger thereto, the outrigger comprising: a support; a lever pivotally connected to the support; and spring means cooperating between the support and the lever to urge the lever to pivot towards a first position, the outrigger further comprising means for adjustably affixing the support the said a mounting assembly.

By providing a means for adjustably affixing the support a mounting assembly, the position of the outrigger can suitably be adjusted in-situ, thus facilitating the initial set-up of a dynamic splint incorporating the outrigger, and additionally or alternatively, facilitating the subsequent adjustment of the outrigger or dynamic splint. Such a configuration may significantly reduce the set-up time and complexity of a dynamic splint incorporating an outrigger according to the invention.

The pivoting connection between the outrigger's support and the lever may be of any suitable type, such as, for example, a pintle hinge assembly, a clip hinge, or a line of weakness formed at an intersection between an integrally formed lever and support, any in any case, a moveable connection allowing the lever to pivot relative to the support. The lever is preferably detachably affixable to the support to enable it to be replaced by other levers of differing dimensions.

The lever pivots relative to the support and a spring means is provided to urge the lever to pivot towards a first position. The spring means may take any suitable form. In one possible embodiment, the spring means comprises a resiliently deformable member, such as a coil spring or an elasticated tie, connected to the lever and support at positions spaced apart from the pivot point. The spring means may comprise may be made from, or comprise, a shape-memory material, such as Nitinal (RTM), that is to say, a material that does not obey Hooke's law throughout its entire range of movement. The spring force may be adjustable, for example, by providing alternative connection points for the spring means or by providing a slider assembly that allows the distance between the connector points for a spring means to be adjusted.

In another possible embodiment of the invention, a first one of the lever and support comprises a cam surface arranged to cooperate with a resilient abutment of a second one of the lever and support, such that the abutment cooperates with the cam surface to urge the lever towards the first position.

The outrigger comprises means for adjustably and/or detachably affixing the support a mounting assembly. Such means may comprise channel or groove in a first one of the support and mounting assembly that cooperates with a complementarily-shaped appendage of a second one of the support and mounting assembly that permits relative movement or sliding of the two. Locking means, for example, a grub screw, is suitably provided for releasably locking the relative positions of the support and mounting assembly, or an adhesive may be used for permanently, or semi-permanently, locking the relative positions of the support and mounting assembly. In one possible embodiment of the invention, either or both of the support and mounting assembly may be manufactured from a resiliently deformable, or elastomeric, material, to facilitate a relative tight friction or interference fit between the respective parts for semi-permanently locking their relative positions.

The mounting assembly is suitably affixable, in use, to a patient's body and will suitably be ergonomically shaped to fit the appropriate body part. The mounting assembly may take the form of a strap that can be wrapped around a body part adjacent a joint to be treated, which strap, for example in the case of a finger support, may take the form of a glove or hand/wrist bandage. The mounting assembly may additionally comprise a stiffening means, for example, a relatively rigid plate.

Various embodiments of the invention shall now be described, by way of example only, with reference to the accompanying drawings in which:
Figure 1 is a perspective, partially cut-away and exploded view of a first embodiment of a dynamic splint in accordance with the invention;
Figure 2 is a perspective view of a first alternative outrigger for the dynamic splint of Figure 1;
Figure 3 is a perspective view of an alternative mounting assembly for the dynamic splint of Figure 1 or the outrigger of Figure 2;
Figure 4 is a perspective view of a second alternative outrigger for the dynamic splint of Figures 1, 2 or 3;
Figures 5, 6 and 7 are a sequence showing a third alternative embodiment of an outrigger for the dynamic splint of Figures 1, 2 or 3;
Figure 8 is a perspective view of a dynamic splint incorporating an outrigger as shown in Figures 5, 6 and 7; and
Figure 9 is a schematic cross-section through an outrigger and the channel of Figure 8.

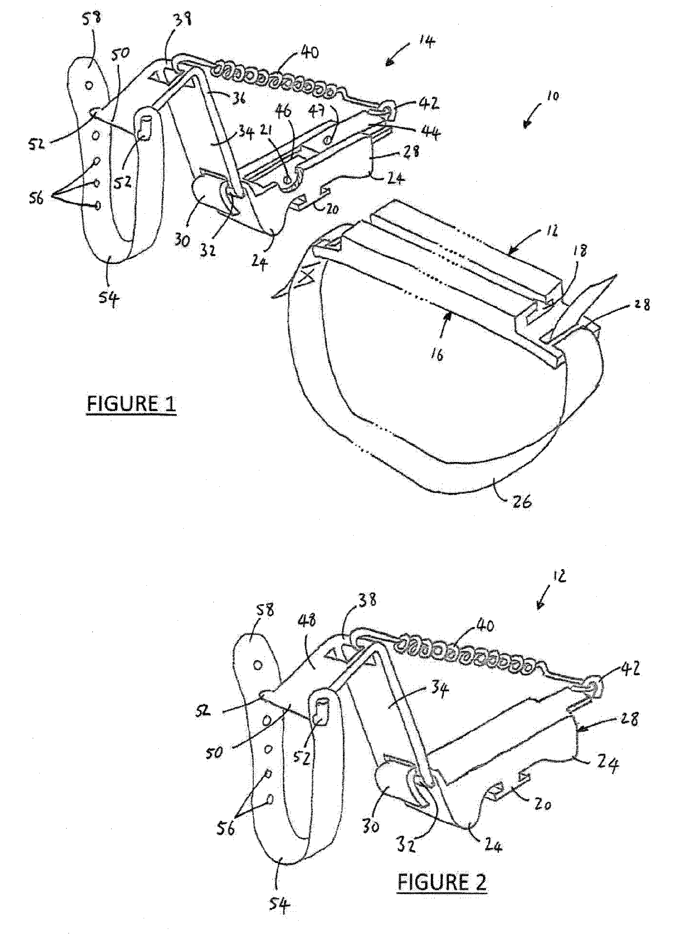

In Figure 1, a dynamic splint 10 comprises a mounting assembly 12 and an outrigger 14 that is detachably affixable to the mounting assembly 12. In many cases, such as when used for supporting fingers, the dynamic splint will be adapted to carry a plurality of outriggers 14 corresponding to the number of joints to be treated.

The mounting assembly 12 comprises a channel member 16 proving an undercut channel 18 into which a correspondingly-shaped runner formation 20 integrally formed with the base 22 of an outrigger 14 can slide. The runner formation 20 is adapted to slide into the channel 18 to a desired location, and is stabilised against rocking by a pair of abutment members 24 that protrude down from the base 22 of the outrigger 14 to contact, in use, the wearer's body (not shown) on opposite sides of the channel member 16. The channel member 16 is manufactured from an elastomeric material, such as silicone rubber, so that it can conform to the shape of an underlying body part (not shown) for a comfortable fit. By manufacturing the channel member 16 from an elastomeric material, it will be appreciated that the dimensions of the channel 18 can be made slightly smaller than those of the runner formation 20 so that the channel member 16 has to stretch to fit the runner 20 thus providing a convenient friction fit to retain the outrigger 14 at a desired position along its length. Such a configuration ready permits adjustment of the location of the outrigger or outriggers 14, for example, so that they can be aligned, in the case of a finger splint, with the wearer's knuckles.

A supplementary locking grub screw 21 is also provided in the outrigger, which can be screwed down into the base of the channel 18 to semi-permanently lock the outrigger 14 in position.

The channel member 16 is held in place by an adjustable, elasticated strap 26, which passes through holes 28 provided at opposite ends of the channel member 16. The strap shown in Figure 1 is of a very simple design, but it will be appreciated that the strap 26 could be replaced by straps of differing designs depending on the ergonomics of the body part (not shown) to which the splint 10 is affixed.

The outrigger 14 comprises a support member 28 which is formed as an injection moulded plastics block. The runner formation 20 is integrally formed with the support to provide a unitary assembly. One end of the support member 20 comprises an inverted-U shaped formation 30 that provides a resilient connector that engages with a cross-bar 32 integrally formed at the base of a lever 34. The lever 34 can thus be clipped onto the support member 28 and is free to pivot about the cross-bar 32, which pivot axis, it will be noted, is offset relative to the pivot axis of a joint (not shown) being treated.

The free end 36 of the lever 34 comprises an additional, upper cross-bar 38 to which one end of a helical coil spring 40 is connected via hook portion (not clearly visible) formed at the end of the spring 40. The other end of the spring 40 is similarly connected to a hook 42 that is integrally formed with a slider 44 that is arranged to slide within a channel 46 formed in the upper surface of the support member 28, and which can be locked in place by a grub screw 47. By such an arrangement, the tension in the spring and/or the neutral position of the lever 34 can be adjusted by adjusting the position of the slider 44 relative to the support member 28.

The free end 36 of the lever 34 additionally comprises a perpendicularly-extending limb portion 48, which carries, at its free end 50, a pair of lugs 52 to which a support sling 54 can be affixed by passing the lugs 52 one of a selection of through holes 56 in the sling 54. Once properly adjusted, the free ends 58 of the sling 54 can be trimmed off, if desired.

The dynamic splint 10 thus addresses certain problems associated with known dynamic splints, for example:
By providing an adjustable mounting assembly 12, the need to form a mould of a wearer's body part is removed, which can considerably reduce initial fitting and set-up times. Also, by providing an adjustable connection between the outrigger(s) 14 and the mounting assembly 12, the need to glue, reposition and re-glue the outriggers to the mounting assembly is removed, thus greatly simplifying the initial fitting of a dynamic splint. Further, by allowing the location of the outrigger(s) to be adjusted, allowing the lever(s) to be detachable, by enabling the spring tension and/or neutral point to be adjustable, initial set-up and subsequent re-adjustment of the dynamic splint is greatly facilitated. It will be appreciated that the dynamic splint of the invention still affords a great deal of flexibility and configurability in terms of set up and adjustment, without compromising on utility, ergonomics or efficacy.

In many situations, the initial set-up parameters of a dynamic splint are standardised, or lie with a small range, and so the need for all of the adjustment capabilities of the device shown in Figure 1 may not be necessary or desirable. In such a situation, a simplified version of the outrigger, such as that shown in Figure 2, may suffice.

In Figure 2, the outrigger 14 is substantially the same as that shown in Figure 1 and identical reference signs have been used to identify identical features to avoid repetition and to aid intelligibility. The main difference between the simplified version of the outrigger 14 shown in Figure 2 and that of Figure 1 is the omission of the slider 44 for adjusting the spring 40 tension and neutral position. In this embodiment of the invention, these parameters can still be adjusted, albeit by substituting the spring 40 for one of a different length or Hooke constant: the spring 40 being detachably affixable to the support member 28 and the lever 34 by a hook 42 and upper cross-bar 38, respectively. In addition, it will be noted that the lever 34 of the simplified embodiment can still be interchanged by un-clipping it and replacing it with another lever of differing dimensions.

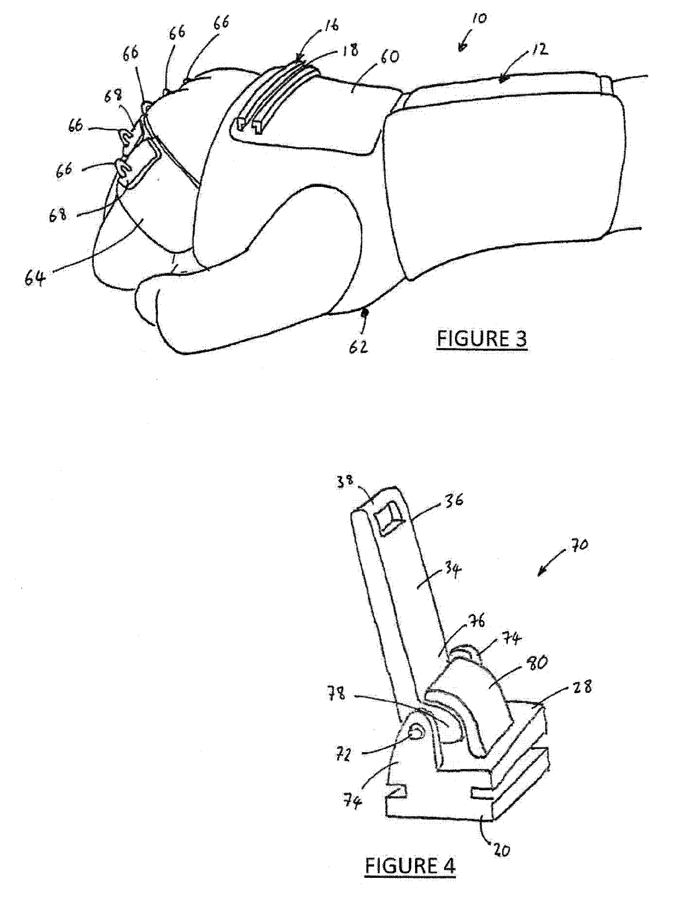

Figure 3 shows an alternative type of mounting assembly 12 for outriggers (not shown) of the type previously described. The mounting assembly 12 comprises a channel member 16 functionally similar to that previously described, the channel member 16 being integrally formed with a rigid or semi-rigid support plate 60 that is stitched to the exterior surface (in the illustrated embodiment) of an elasticated wrist support 62. It will be appreciated that the wrist support 62 shown could be substituted for another type of support depending on the ergonomic requirements of the wearer, for example, the dynamic splint 10 could be fitted to a knee, ankle, elbow or other joint support. The wrist support 62 comprises a web of flexible elasticated textile that can be wrapped around a body part (in this case the hand and wrist) adjacent a joint to be treated (in this case a knuckle joint). The wrist support comprises a hook and loop type fastener that enables a wearer to adjust the tightness of the support 62 to personal requirements.

The provision of an integrally-formed support plate serves to stabilise the channel member 16 on the back of the user's hand, thus allowing the tension in the spring 40 to be transmitted between the lever 34 and the wearer's body in a predictable and controlled manner.

It will also be noted, in Figure 3, that the simple slings 54 shown in Figures 1 and 2 have been substituted for tubular finger ring portions 64 that are made of a relatively inextensible textile and which have eyelets 66 formed on their intended upper surfaces that connect to the finger ring portions 64 via small support plates 68. The eyelets 66 can be connected to the upper cross-bars 38 of the levers 34 previously described, by relatively inextensible linkages to allow the spring force to be used to urge the fingers toward extension via the levers 34.

Figure 4 shows a yet further alternative embodiment of the outriggers 14 in which the spring 40 previously described has been replaced by a sprung cam assembly 70. A known problem, associated with existing dynamic splints, such as that described in EP1071385, is that there is a tendency for the outriggers and/or springs to snag on foreign objects, especially clothing, and most especially sleeves of clothing when they are being put on, or removed, by a user. In the embodiment of Figure 4, the coil-sprung arrangement has been replaced by a cam-type spring arrangement, which reduces the likelihood of snagging on foreign objects.

The outrigger 14 of Figure 4 comprises a support member 28 having an integrally formed runner 20, and a pivotally connected lever 34, similar to that previously described. In this example, the lever 34 is pivotally connected to the support member 28 by a pair of outwardly extending bosses 72 that engage with through holes or recesses formed in a clevis 74 formed integrally with the support element 28. The lever 34 can be removed and replaced by prising the clevis 74 apart to free the bosses 72 from the through holes or recesses in an manner that will be readily apparent to the skilled person.

The lower end 76 of the lever 34 comprises an integrally formed cam portion 78 that extends radially outwardly from the axis of the bosses 72, which cam portion 78 is adapted to slide against and cooperate with, a resiliently deformable cam follower plate 80 formed integrally with the support member 28 of the outrigger 14. The lever 34 is thus urged towards an upright (as shown in the drawings) neutral position by the action of the cam portion 78 on the cam follower 80, but is free to pivot about the axis of the bosses 72 by the cam portion 78 resiliently deforming the cam follower 80. The lever 34 is thus biased towards the neutral position, which effect can be used to provide the therapeutic force required for the effective operation of the dynamic splint. It will be appreciated that the neutral position and the spring force can be selected by the appropriate selection of the geometry of the cam member 78 and the cam follower 80. Specifically, by purposively designing the profile of the cam and cam follower, it is possible to tailor the restorative spring force to be zero at, or through, several angles of rotation, thus enabling the spring force to be matched more precisely to biomechanical requirements.

Further, the embodiment of the invention of Figure 4 has been depicted with a generally upright lever arrangement and with a cam and cam follower located on top of the support. This is merely an exemplary embodiment and has been depicted thus to facilitate direct comparison with the embodiments shown in Figures 1 and 2. Nevertheless, it will be appreciated that the lever and cam could be reconfigured such that the neutral position of the lever is more in-line with the user's hand, meaning that it protrudes upwardly less from the back of the user's hand, and/or the cam could be formed to face downwardly with the cam follower abutting it from below, to provide a flatter overall design that poses a yet lesser snagging hazard.

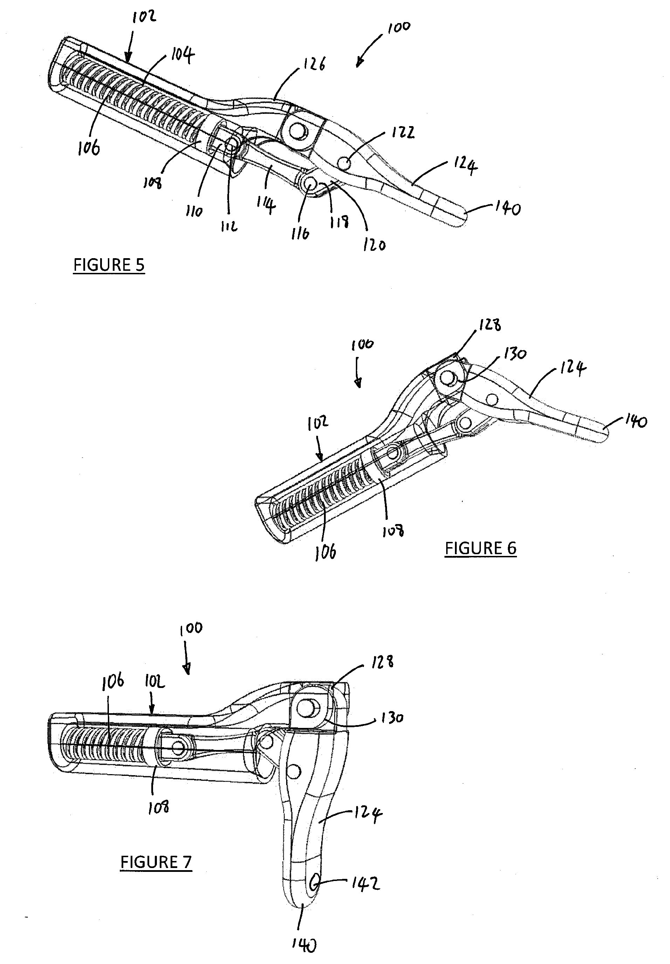

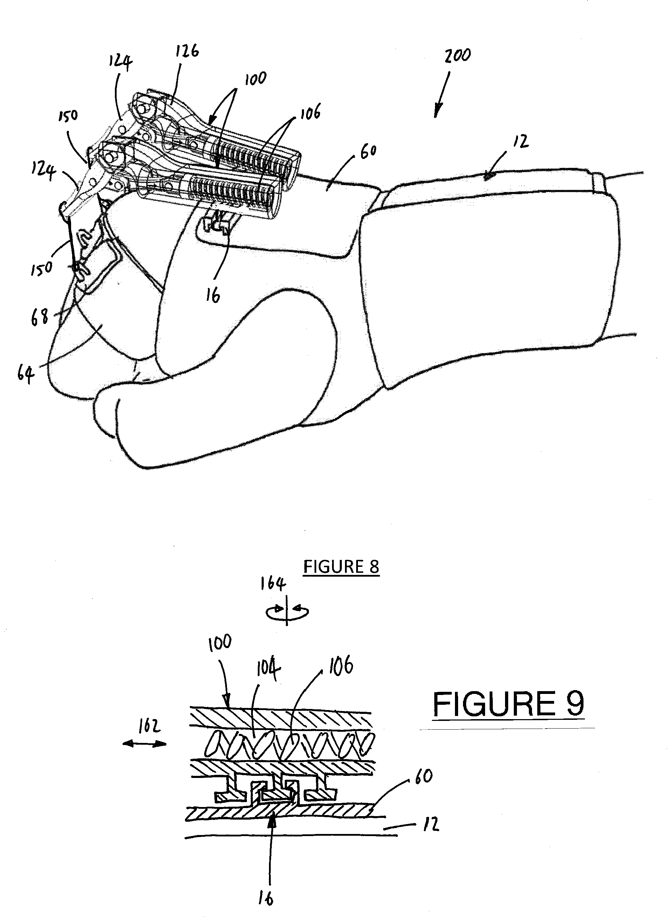

In Figures 5 to 7, another embodiment of an outrigger 100 in accordance with the invention comprises a main body portion 102 moveably affixable, in use, to a support structure 60, such as that shown in Figures 3 and 8. The main body potion 102 comprises a generally cylindrical hollow interior volume 104, which houses a helical compression spring 106. The spring 106 acts upon a disc 108, which is integrally formed with a clevis 110, which pivotally connects to one end 112 of a first linkage 114. The opposite end 116 of the first linkage 114 connects to a first end 118 of a second linkage 120, whose opposite end 122 connects, in turn, to a lever 124. The lever 124 is pivotally connected to an extension 126 extending from the main body portion 102.

As can be best seen in Figures 6 and 7, the extension 126 has a part-rounded 128 and a part squared-off 130 portion which limits the movement of the lever 124 between a fully-extended, straight position (as shown in Figure 5) through an intermediate position (as shown in Figure 6) and to a fully-bent position (as shown in Figure 7).

The lever 124 is biased towards the straight position (as shown in Figure 5) by the action of the spring 106 acting through the two linkages 114, 120 on the lever 124. Because there are two linkages 114, 120, the disc 108 is able to travel linearly along the interior of the hollow interior volume 104 of the main body portion 102 as any vertical offset is accommodated by the relative movements of the linkages 114, 120. Moreover, the force-extension curve, that is to say, the biasing force applied by the spring to the lever at different points of travel, can be tailored by adjusting the length of the spring 106 and the length of the levers. In the illustrated embodiment, the forces are resolved such that the restoring force urging the lever to the extended position (as shown in Figure 5) is substantially the same at all positions. A constant force has been shown to provide positive therapeutic benefits when used in a splinting device because it alleviates fatigue and allows the wearer to use the affected body part, e.g. finger, leg, arm, throughout its entire range of movement, that is to say, without excessive resistance at the extremes of movement and without "force fade" as the affected body part is straightened.

It will be noted that the construction shown in Figures 5 to 7 inhibits losing the spring 106 because the squared-off end 130 of the extension 126 prevents the disc 108 form travelling beyond the opening of the hollow interior volume 104. However, the outrigger 100 can be readily assembled by clipping the linkages together after the spring 106 and disc 108 have been located.

The free end 140 of the lever 124 can be connected to a sling 54, such as that shown in Figures 1 and 2, via a through hole 142, as shown in Figure 7.

In Figure 7, a dynamic splint 200 ain accordance with the invention comprises a mounting assembly 12 for the outriggers of the type shown in Figures 5, 6 and 7 of the drawings. The mounting assembly 12 comprises a channel member 16, which is integrally formed with a rigid or semi-rigid support plate 60 that is stitched to the exterior surface (in the illustrated embodiment) of an elasticated wrist support 62. It will be appreciated that the wrist support 62 shown could be substituted for another type of support depending on the ergonomic requirements of the wearer, for example, the dynamic splint 10 could be fitted to a knee, ankle, elbow or other joint support. The wrist support 62 comprises a web of flexible elasticated textile that can be wrapped around a body part (in this case the hand and wrist) adjacent a joint to be treated (in this case a knuckle joint). The wrist support comprises a hook and loop type fastener that enables a wearer to adjust the tightness of the support 62 to personal requirements.

The provision of an integrally-formed support plate 60 serves to stabilise the channel member 16 on the back of the user's hand, thus allowing the tension in the spring 106 to be transmitted between the lever 124 and the wearer's body in a predictable and controlled manner.

It will also be noted, in Figure 8, that tubular finger ring portions 64 are connected to the levers 124 of the outriggers 100 via length adjustable wires 150.

In Figure 9 it can be seen how the outriggers 100 are connected to the channel 16 of the support plate 60: that is to say, via a set of projections 160. Each outrigger 100 is provided with a number of projections 160 so that the projection 162 of the outrigger beyond the channel 16 can be selected by a user. The projections 160 are also rotatably receivable in the channel 16 to permit the outriggers 100 to pivot or rotate 164 about the projection. This configuration allows a wearer of the device 200 a full range of movement and reduces transverse loading on the affected body part, as might occur if the outriggers 100 were to be rigidly affixed to the channel 16.

The invention is not restricted to the details of the foregoing embodiments, which are merely exemplary of the invention. For example, the shape and configuration of the various elements of the invention may be changed, as might their materials of manufacture. In one alternative embodiment, the finger slings shown in the drawings may be replaced by, or supplemented by, finger-tip engaging supports (similar to rigid or flexible thimbles) that fit over the tips of a wearer's fingers. The invention is not restricted to the field of dynamic finger splints, but may be applied to other joints, for example, but without limitation to, elbow, wrist, knee and ankle joints.

The following statements are not the claims, but relate to various aspects of the invention:
Statement 1. An outrigger suitable for use with a dynamic splint, the outrigger comprising a support, a lever pivotally connected to the support, and spring means cooperating between the support and the lever to urge the lever to pivot towards a first position, wherein the outrigger further comprises means for adjustably affixing the support to a mounting assembly.
Statement 2. An outrigger according to statement 1, wherein the means for adjustably affixing the support to the mounting assembly comprises a channel or groove in a first one of the support and mounting assembly that cooperates with a complementarily-shaped appendage of a second one of the support and mounting assembly.
Statement 3. An outrigger according to statement 1 or statement 2, wherein the means for adjustably affixing the support to the mounting assembly is adapted to permit relative movement or sliding of the two.
Statement 4. An outrigger according to statement 2 or statement 3, further comprising locking means for releasably locking the relative positions of the support and mounting assembly.
Statement 5. An outrigger according to statement 4, wherein the locking means comprises any one or more of the group comprising: a grub screw; an adhesive; a worm screw and a cross-helical screw assembly.
Statement 6. An outrigger according to any preceding statement, wherein either or both of the support and mounting assembly are manufactured from a resiliently deformable, or elastomeric, material.
Statement 7. An outrigger according to statement 6, adapted to provide a relatively tight friction and/or interference fit between the respective parts for semi-permanently locking their relative positions.
Statement 8. An outrigger according to in any preceding statement, wherein the pivoting connection between the lever and the support comprises any one or more of the group comprising: a pintle hinge assembly; a clip hinge; a line of weakness formed at an intersection between an integrally formed lever and support; and a moveable connection allowing the lever to pivot relative to the support.
Statement 9. An outrigger according to any preceding statement, wherein the lever is detachably affixable to the support.
Statement 10. An outrigger according to any preceding statement, wherein the spring means comprises a resiliently deformable member connected to the lever and support at positions spaced apart from the pivot point.
Statement 11. An outrigger according to statement 10, wherein the spring force and/or neutral position is adjustable.
Statement 12. An outrigger according to statement 11, wherein the spring means is connectable to the support or lever at different connection points.
Statement 13. An outrigger according to statement 11 or statement 12, further comprising an adjustable slider to which one end of the spring means is connectable.
Statement 14. An outrigger according to any of statements 1 to 9, wherein a first one of the lever and support comprises a cam surface arranged to cooperate with a resilient abutment of a second one of the lever and support, the abutment being arranged to cooperate with the cam surface to urge the lever towards the first position.
Statement 15. A mounting assembly for a dynamic splint comprising means for adjustably affixing an outrigger thereto, the outrigger comprising: a support; a lever pivotally connected to the support; and spring means cooperating between the support and the lever to urge the lever to pivot towards a first position, the outrigger further comprising means for adjustably affixing the support to the said a mounting assembly.
Statement 16. A mounting assembly according to 15, wherein the outrigger comprises an outrigger according to any of statements 1 to 14.
Statement 17. A mounting assembly according to statement 15 or statement 16, comprising a strap that can be wrapped around a body part adjacent a joint to be treated.
Statement 18. A mounting assembly according to statement 17, wherein the strap comprises any one or more of the group comprising: a glove; a hand wrap; a wrist wrap; a knee support; an ankle support and an elbow support.
Statement 19. A mounting assembly according to any of statements 15 to 18, further comprising a stiffening means.
Statement 20. A mounting assembly according to statement 19, wherein the stiffening means comprises a relatively rigid plate.
Statement 21. A dynamic splint comprising a mounting assembly according to any of statements 15 to 20 and an outrigger according to any of statements 1 to 14.
Statement 22. A dynamic split according to statement 21, wherein the lever additionally comprises a perpendicularly-extending limb portion, which carries, at its free end, a pair of lugs to which a support sling is affixable.
Statement 23. A kit of parts for forming a dynamic splint comprising: a mounting assembly according to any of statements 15 to 20 and an outrigger according to any of statements 1 to 14.
Statement 24. A kit of parts according to statement 23, further comprising a set of substitutable components from the group comprising: a set of levers of differing lengths; a set of springs of differing lengths; a set of springs of differing Hooke moduli; a set of support members of differing dimensions; a set of mounting assemblies adapted to fit different, and differently-sized body parts; and a set of slings adapted to fit different, and differently-sized body parts.
Statement 25. An outrigger, mounting assembly, dynamic splint or kit of parts substantially as hereinbefore described, with reference to, and as illustrated in, the accompanying drawings.

## Claims

1. An outrigger (14, 100) suitable for use with a dynamic splint (200), the outrigger (100) comprising a support (22, 102), a lever (34, 124) pivotally connected to the support (22, 102), and a spring means (40, 106) cooperating between the support (22, 102) and the lever (34, 124) to urge the lever (34, 124) to pivot towards a first position, wherein the outrigger (14, 100) further comprises means (20) for adjustably affixing the support (22, 102) to a mounting assembly (12, 60).

2. The outrigger (14, 100) of claim 1, wherein the means (20) for adjustably affixing the support to the mounting assembly (12, 60) comprises a channel or groove (16) in a first one of the support and mounting assembly (12, 60) that cooperates with a complementarily-shaped appendage (20) of a second one of the support and mounting assembly (12, 60).

3. The outrigger (14, 100) of claim 1 or claim 2, wherein the means (20) for adjustably affixing the support to the mounting assembly (12, 60) is adapted to permit any one or more of the group comprising: relative movement of the two; relative sliding of the two; relative rotation of the two (164); and relative extension or retraction of the two (162).

4. The outrigger (14, 100) of claim 1, 2 or 3, further comprising locking means (21) for releasably locking the relative positions of the support (22, 102) and mounting assembly (12, 60).

5. The outrigger (14, 100) of claim 4, wherein the locking means (21) comprises any one or more of the group comprising: a grub screw (21); an adhesive; a worm screw; a cross-helical screw assembly; and an interference fit between the respective parts (16, 18, 20) for semi-permanently locking their relative positions.

6. The outrigger (14, 100) of any preceding claim, wherein the spring means (40, 106) comprises any one or more of the group comprising: a resiliently deformable member (40) connected to the lever (34, 124) and support (22, 102) at positions spaced apart from the pivot point (32); a first one of the lever (34, 124) and support (22, 102) comprising a cam surface (78) arranged to cooperate with a resilient abutment (80) of a second one of the lever (34, 124) and support (22, 102), the abutment (80) being arranged to cooperate with the cam surface (78) to urge the lever (34, 124) towards the first position; a compression spring (106) acting on the lever (124) via a moveable linkage (114, 120); and a compression spring (106) acting on the lever (124) via series of moveably interconnected linkages (114, 120).

7. The outrigger (14, 100) of any preceding claim, wherein the spring force and/or neutral position of the spring means (40, 106) is adjustable by any one or more of the group comprising: the spring means comprising a spring (40) interconnectable between the support (22) and lever (34) at different connection points; and the spring means comprising a spring (40) and further comprising an adjustable slider (44) to which one end of the spring (40) is connectable.

8. The outrigger (14, 100) of any preceding claim, wherein the lever (34) comprises a perpendicularly-extending limb portion (50), which carries, at its free end, a pair of lugs (52), and a support sling (54) affixable to the lugs (52).

9. A mounting assembly (12, 60) for a dynamic splint (10) comprising a connector (20) for adjustably affixing an outrigger (14) thereto, the outrigger (14, 100) comprising: a support (22, 102); a lever (34, 124) pivotally connected to the support (22, 102); and spring means (40, 106) cooperating between the support (22, 102) and the lever (34, 124) to urge the lever (34, 124) to pivot towards a first position, the outrigger (14, 100) further comprising the connector (20) for adjustably connecting the support (22, 102) to the said a mounting assembly (12, 60).

10. The mounting assembly (12, 60) of claim 9, further comprising a strap (62) that can be wrapped around a body part adjacent a joint to be treated.

11. The mounting assembly (12, 60) of claim 10, wherein the strap (62) comprises any one or more of the group comprising: a glove; a hand wrap; a wrist wrap; a knee support; an ankle support and an elbow support.

12. The mounting assembly (12, 60) of claim 11, further comprising a stiffener.

13. The mounting assembly (12, 60) of claim 12, wherein the stiffener comprises a relatively rigid plate.

14. A dynamic splint (10) comprising the mounting assembly (12, 60) of any of claims 9 to 13 and the outrigger (14, 100) of any of claims 1 to 8.

15. A kit of parts for forming a dynamic splint comprising: the mounting assembly (12, 60) of any of claims 9 to 13 and the outrigger (14, 100) of any of claims 1 to 8, said kit further comprising a set of substitutable components from the group comprising: a set of levers (34, 124) of differing lengths; a set of springs (40, 106) of differing lengths; a set of springs (40 106) of differing Hooke moduli; a set of support members (22, 102) of differing dimensions; a set of mounting assemblies (12, 60) adapted to fit different, and differently-sized body parts; and a set of slings (54) adapted to fit different, and differently-sized body parts.
